# EUROPEAN PATENT APPLICATION

(11) **EP 2 574 351 A1**
(43) Date of publication of application: **03.04.2013**
(21) Application number: 12185030.9
(22) Date of filing: 19.09.2012
(51) Int. Cl.: A61L 29/16, A61M 25/00

(54) **Plasma-treated dialysis catheter cuff**

(30) Priority: 29.09.2011 US 201113248436
(71) Applicant: Covidien LP, Mansfield, MA 02048 (US)
(72) Inventor: Sheu, Min-Shyan, Chelmsford, MA Massachusetts 01824 (US)
(74) Representative: Gray, James

(57) **Abstract**

A catheter is disclosed. The catheter includes an elongated tubular body defining a longitudinal axis and extending to a distal end thereof, the tubular body having at least one lumen and a cuff disposed around the tubular body configured to contact tissue, the cuff formed from a plasma-treated material having enhanced tissue ingrowth properties.

## Description

### BACKGROUND

### 1. Technical Field

The present disclosure relates to medical catheters, and more particularly to catheters having a plasma-treated cuff having enhanced tissue ingrowth properties.

### 2. Background of the Related Art

In certain medical treatments, it is desirable to establish long term vascular access to a specific desired interior body site for purposes of administering fluids to and/or for removing bodily fluids from the body for purification, testing, monitoring, or disposal.

Particularly in the case of administering fluids to, or removing fluids from, the body continuously or periodically over an extended time period, it is known in the medical arts to use what are known as "permanent" catheterization techniques employing subcutaneous-implanted devices such as hemodialysis catheters and tunneled central venous catheters (CVCs) for durations ranging from a few weeks to years. Examples of such subcutaneous-implanted and related medical devices are found in U.S. Patent Nos. 7,141,035 and 7,777,605, the entire contents of which are incorporated by reference herein. Examples of therapeutic regimens requiring such long term continuous or periodic access to a specific internal body location include parenteral feeding, chemotherapy, antibiotic administration, dialysis, and the like.

Typically, catheters are tubular, flexible medical devices, which have one or more lumens, e.g., dual lumens or triple lumens. Multiple lumen catheters facilitate bidirectional fluid flow whereby one lumen, for example, performs withdrawal of blood and the other lumen reintroduces treated blood to a vessel. During an exemplary hemodialysis procedure, a multiple lumen catheter is inserted into a body and blood is withdrawn through an arterial lumen of the catheter. This blood is supplied to a hemodialysis unit which dialyzes, or cleans, the blood to remove waste and excess water. The dialyzed blood is returned to the patient through a venous lumen of the catheter. Typically, the venous lumen is separated from the arterial lumen by an inner catheter wall, called a septum.

In certain classes of medical catheter devices, for instance peritoneal dialysis catheters, promoting epidermal tissue growth around a cuff or lip of the device can provide a number of advantages. The epidermis is both the body's natural barrier to infection and the habitat of the largest reservoir of microorganisms causing catheter-related infection. Trauma to the epidermis, such as the incision required for insertion of catheters into the body, sets in motion a series of physiological mechanisms designed to heal the trauma and to re-establish the skin's capacity to prevent infection. Insight into these natural mechanisms, and equipping catheters with cuff technology at the incision site to facilitate the successful completion of these natural defense mechanisms, provide for an effective strategy to reduce catheter-related morbidity as well as cost. Thus, it is desirable to provide for a catheter cuff having improved tissue ingrowth performance.

### SUMMARY

The present disclosure provides for a catheter. The catheter includes an elongated tubular body defining one or more lumens and a longitudinal axis, the tubular body having a distal end and a proximal end. The catheter also includes a cuff disposed around the tubular body configured to contact tissue, the cuff formed from a plasma-treated material.

In embodiments, a method for plasma-treating a catheter cuff is also disclosed. The method includes supplying a mixture of oxygen and at least one hydrocarbon gas into a plasma chamber; igniting the mixture to form a plasma; and directing the plasma over a catheter cuff.

In further embodiments, a method for plasma-treating a catheter cuff is further disclosed. The method includes supplying a mixture of oxygen and methane into a plasma chamber at a combined flow rate from about 15 sccm to about 40 sccm; igniting the mixture to form a plasma; and directing the plasma for about 1 minute to about 5 minutes over a catheter cuff.

### BRIEF DESCRIPTION OF THE DRAWINGS

Various embodiments of the present disclosure are described herein below with references to the drawings, wherein:
Fig. 1 shows a perspective, partially cross-sectional view of a catheter according to the present dislcosure;
Fig. 2 shows a front, cross-sectional view taken along section lines 2-2 of Fig. 1;
Fig. 3 shows photographs of rats implanted with the catheter of Fig. 1;
Fig. 4 shows photographs of cross-sectional slices of plasma-treated and untreated catheter cuffs from the catheters implanted into the rats after seven days;
Fig. 5 shows a bar graph of cell infiltration into the catheter cuffs of Fig. 4;
Fig. 6 shows a bar graph of cell density within the catheter cuffs of Fig. 4;
Fig. 7 shows a bar graph of collagen production within the catheter cuffs of Fig. 4;
Fig. 8 shows a bar graph of the tissue adhesion forces of the catheter cuffs of Fig. 4; and
Fig. 9 shows a graph of tissue ingrowth versus time of the catheter cuffs of Fig. 4.

### DETAILED DESCRIPTION OF THE EMBODIMENTS

The exemplary embodiments of the catheter and methods of use disclosed are discussed in terms of medical catheters for the administration of fluids (withdrawal, introduction, etc.) to and/or from the body. The catheter is advantageously configured to facilitate reversible fluid flow between lumens thereof. It is envisioned that the present disclosure may be employed with a range of catheters, such as, for example, hemodialysis, peritoneal, infusion, PICC, CVC, and port, and catheter applications including surgical, diagnostic and related treatments of diseases, and body ailments of a subject. It is further envisioned that the principles relating to the catheter disclosed include employment with various catheter related procedures, such as, for example, hemodialysis, cardiac, abdominal, urinary, and intestinal, in chronic, and acute applications.

In the discussion that follows, the term "proximal" will refer to the portion of a structure that is closer to a practitioner, while the term "distal" will refer to the portion that is further from the practitioner. As used herein, the term "subject" refers to a human patient or other animal. According to the present disclosure, the term "practitioner" refers to a doctor, nurse or other care provider and may include support personnel.

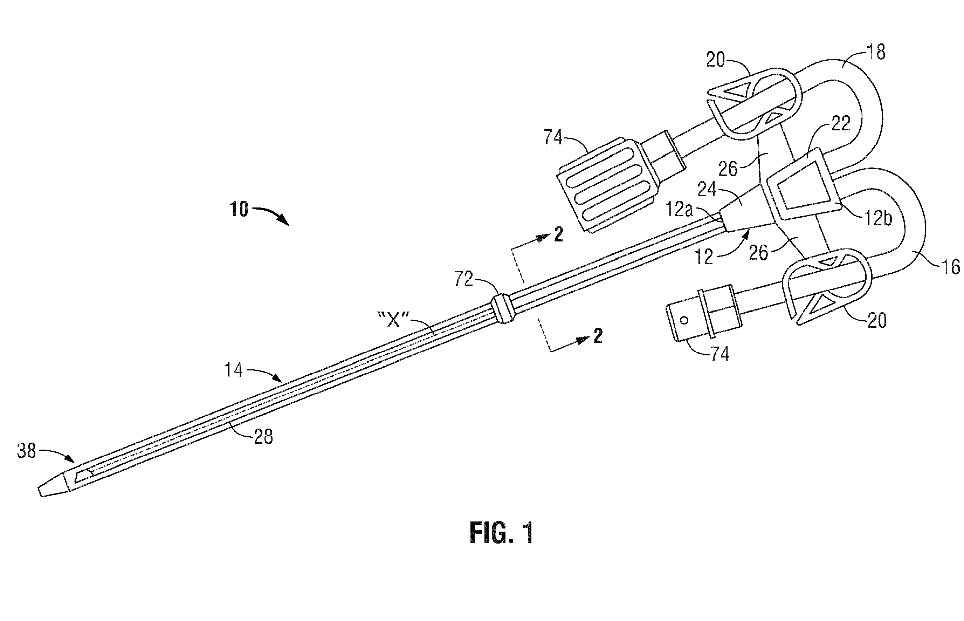

Referring now to the drawings, wherein like components are designated by like reference numerals throughout the several views, Fig. 1 illustrates an exemplary hemodialysis catheter assembly 10 in accordance with the principles of the present disclosure. The catheter assembly 10 includes a catheter hub 12 having respective distal and proximal ends 12a, 12b, an elongated catheter member 14 that extends distally from the catheter hub 12, and first and second extension tubes 16, 18 that extend proximally from the catheter hub 12. The catheter assembly 10 may be provided with the hub 12 integrally formed with the catheter member 14. Alternatively, the hub 12 may be configured for attachment to the catheter member 14 after catheter placement into a patient by the clinician. The catheter assembly 10 further includes a pair of clamps 20 that are positionable about the extension tubes 16, 18. Each clamp 20 is movable from an open position to a substantially closed position to compress a corresponding extension tube 16, 18, and thereby inhibit fluid flow through the extension tubes 16, 18. Each of the extension tubes 16, 18 includes a connector 74 at its proximal end for coupling to a hemodialysis apparatus.

The catheter hub 12 is configured and dimensioned for grasping by a clinician, and includes a proximal (trailing) housing section 22 that is positioned adjacent the extension tubes 16, 18, and a distal (leading) housing section 24 that is positioned adjacent the catheter member 14. The proximal housing section 22 is adapted to respectively receive the first and second extension tubes 16, 18 in secured relation. For example, the extension tubes 16, 18 may be secured within respective extension conduits (not shown) of the catheter hub 12 through the employ of an interference or frictional fit, cements, adhesives, or in any other suitable manner. The distal housing section 24 of the catheter hub 12 defines a central opening (not shown) that is configured and dimensioned to receive the catheter member 14 in secured relation, such as through the employ of an interference or frictional fit, cements, adhesives, or in any other suitable manner.

In the embodiment of the catheter assembly 10 illustrated in Fig. 1, the catheter hub 12 further includes a pair of opposed wings 26 that depend outwardly from the catheter hub 12. The wings 26 serve as a surface about which one or more sutures (not shown) may be secured to fix the catheter hub 12 relative to the patient. Alternatively, the wings 26 or the catheter hub 12 may include an annular groove (not shown) in an outer surface thereof that is configured and dimensioned to receive a suture(s), in which case, the suture(s) may be positioned within the annular groove, and subsequently secured to the patient.

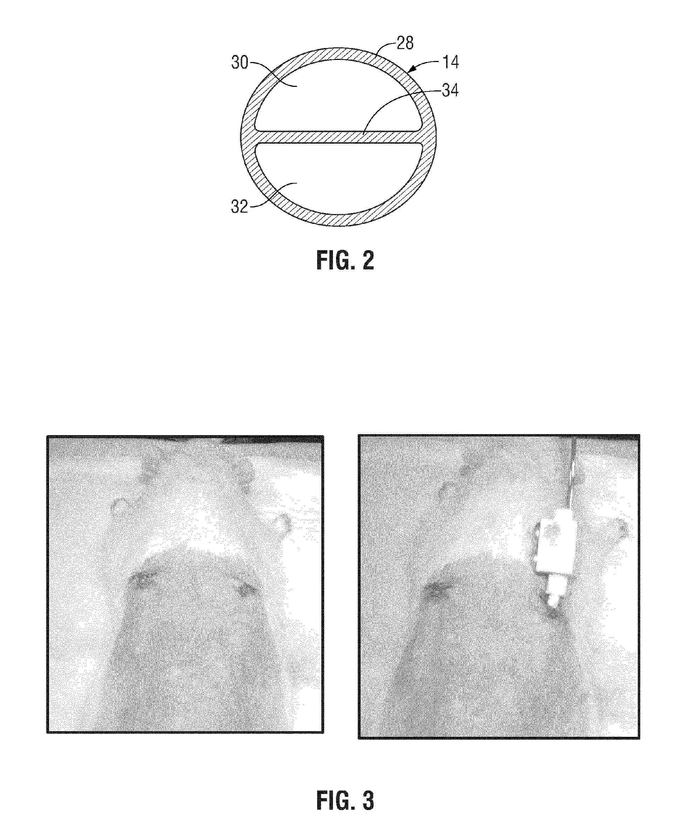

Referring to Figs. 1 and 2, the catheter member 14 includes an outer wall 28, and defines a longitudinal axis "X." It is envisioned that the outer wall 28 of catheter member 14 may include reinforcing material to increase the stability and rigidity thereof, if necessary or desired. As shown in Fig. 2, in one embodiment of the disclosure, the catheter member 14 may assume a dual lumen configuration including respective first and second internal lumens 30, 32 that are separated by a septum wall 34, which may or may not extend the length the catheter member 14. In this embodiment, the respective first and second longitudinal lumens 30, 32 are each configured and dimensioned for fluid communication between proximal and distal ends of the catheter member 14, and may include any cross-sectional configuration suitable for this intended purpose, including but not limited to oblong, kidney-shaped, D-shaped, circular, pie shaped, or the like. While it is envisioned that either lumen 30, 32 may function as the intake (arterial) lumen or the return (venous) lumen, throughout the following discussion, the lumen 30 will be referred to as the venous lumen and the lumen 32 will be referred to as the arterial lumen. Although illustrated as side-by-side in orientation in Fig. 2, the lumens 30, 32 may also be positioned in coaxial relation.

The catheter member 14 also includes a leading (distal) end 38 with a catheter tip member 40 integrally formed therewith, or mounted thereto, that is advantageously configured and dimensioned to facilitate initial insertion into body tissue. In embodiments, the tip member 40 may be an occlusion resistant tip as disclosed in a commonly-owned U.S. Patent Nos. U.S. Patent Nos. 7,141,035 and 7,777,605, the entire contents of which are incorporated by reference herein.

With reference to Fig. 1, the catheter member 14 also includes a cuff 72 disposed about the catheter member 14. The cuff 72 provides for tissue ingrowth subcutaneously at the implantation site of the catheter member 14 into the patient for long-term securing of the catheter assembly 10 in an indwelling position. Tissue ingrowth in and around the cuff 72 physically secures the catheter member 14 within the patient and provides additional protection against infection. Without being limited by any particular theory, it is believed that formation of fibrous tissue around the cuff 72 acts as a barrier in prevention of bacteria and other foreign materials and organisms from entering through the implantation site. Thus, rapid tissue ingrowth around the cuff 72 is desired.

The cuff 72 may made of polyethylene terephthalate sold under the name DACRON™ by Invista of Wichita, KS. However, the cuff 72 may be formed from any suitable biocompatible material including absorbable and non-absorbable materials. As used herein, the term "absorbable" includes both biodegradable and bioresorbable materials and denotes materials that decompose, or lose structural integrity under body conditions (e.g., enzymatic degradation, hydrolysis) or are broken down (physically or chemically) under physiologic conditions in the body (e.g., dissolution) such that the degradation products are excretable or absorbable by the body.

Suitable absorbable materials may include polymers such as aliphatic polyesters; polyamides; polyamines; polyalkylene oxalates; poly(anhydrides); polyamidoesters; copoly(ether-esters); poly(carbonates) including tyrosine derived carbonates; poly(hydroxyalkanoates) such as poly(hydroxybutyric acid), poly(hydroxyvaleric acid), and poly(hydroxybutyrate); polyimide carbonates; poly(imino carbonates) such as poly (bisphenol A-iminocarbonate and the like); polyorthoesters; polyoxaesters including those containing amine groups; polyurethanes, and combinations thereof.

Other suitable biodegradable polymers may include, but are not limited to, poly(amino acids) including proteins such as collagen (I, II and III), elastin, fibrin, fibrinogen, silk, and albumin; peptides including sequences for laminin and fibronectin (RGD); polysaccharides such as hyaluronic acid (HA), dextran, alginate, chitin, chitosan, and cellulose; glycosaminoglycan; gut; and combinations thereof.

Suitable non-absorbable materials which may be employed in the present disclosure may include polyolefins such as polyethylene (including ultra high molecular weight polyethylene) and polypropylene including atactic, isotactic, syndiotactic, and blends thereof; polyethylene glycols; silicone rubber; silicones and polysiloxanes such as polydimethylsiloxane and polydiphenylsiloxane; polyethylene oxides; ultra high molecular weight polyethylene; copolymers of polyethylene and polypropylene; polyisobutylene and ethylene-alpha olefin copolymers; fluorinated polyolefins such as fluoroethylenes, fluoropropylenes, fluoroPEGSs, and polytetrafluoroethylene; polyamides such as nylon, Nylon 6, Nylon 6,6, Nylon 6,10, Nylon 11, Nylon 12, and polycaprolactam; polyamines; polyimines; polyesters such as polyethylene terephthalate, polyethylene naphthalate, polytrimethylene terephthalate, and polybutylene terephthalate; polyethers; polybutester; polytetramethylene ether glycol; 1,4-butanediol; polyurethanes; acrylic polymers; methacrylics; vinyl halide polymers and copolymers, such as polyvinyl chloride; polyvinyl alcohols; polyvinyl ethers such as polyvinyl methyl ether; polyvinylidene halides such as polyvinylidene fluoride and polyvinylidene chloride; polychlorofluoroethylene; polyacrylonitrile; polyaryletherketones; polyvinyl ketones; polyvinyl aromatics such as polystyrene; polyvinyl esters such as polyvinyl acetate; copolymers of vinyl monomers with each other and olefins, such as ethylene-methyl methacrylate copolymers; acrylonitrile-styrene copolymers; ABS resins; ethylene-vinyl acetate copolymers; alkyd resins; polycarbonates; polyoxymethylenes; polyphosphazine; polyimides; epoxy resins; aramids; rayon; rayon-triacetate; spandex; silicones; and copolymers and combinations thereof.

In certain embodiments, both absorbable and non-absorbable materials may be employed to form the cuff 72. The cuff 72 may include a multi-layer structure of alternating absorbable and non-absorbable materials. In further embodiments, the cuff 72 may be formed from a non-woven (e.g., felt-like) or porous material.

The present disclosure provides for a system and method for treating the cuff 72 with a plasma to modify the material of the cuff 72 to render the cuff 72 more susceptible to faster tissue ingrowth. The term "plasma" as used herein refers to any matter in which a substantial portion thereof is ionized and is in gaseous form.

In embodiment, plasma may be generated using electrical energy that is delivered to ionizable media. Electrical energy may be delivered as alternating current (AC) electricity, in either continuous or pulsed modes, at a frequency from about 0.1 MHz to about 2,450 MHz and in another embodiment from about 1 MHz to about 160 MHz, using appropriate generators, electrodes, and antennas. Average power delivered to the ionizable media may be from about 10 Watts (W) to about 1000 W, and in embodiments may be from about 50 W to about 200 W.

Ionizable media may be any suitable composition, which may be in gaseous, liquid, or solid state that forms plasma when ionized. Liquid or solid matter may be atomized or nebulized using a nebulizer, a microfluidic device, a piezoelectric pump, an ultrasonic vaporizer, and the like. Examples of suitable ionizable media include, but are not limited to, argon, helium, neon, krypton, xenon, radon, carbon dioxide, nitrogen, hydrogen, oxygen, hydrocarbon gases, such as methane, ethane, propane, butane, and combinations thereof.

The ionizable media may be delivered into a plasma chamber at a flow rate from about 1 standard cubic centimeters per minute (sccm) to about 100 sccm, and in embodiments may be from about 5 sccm to about 35 sccm. If multiple gases are used, each may be delivered at any desired ratio by varying the flow rate for each gas. The pressure within the plasma chamber may be from about 50 milliTorr (mTorr) to about 2000 mTorr, and in embodiments may be from about 500 mTorr to about 1000 mTorr. In embodiments, the plasma may be applied to the cuff 72 for about 10 seconds to about 20 minutes, and in embodiments for about 1 minute to about 5 minutes. Alternately, other plasma application time periods are envisioned.

In embodiments, the plasma may be formed by ionization of a mixture of oxygen and one or more hydrocarbon gases. Oxygen may be supplied at a flow rate from about 1 sccm to about 10 sccm, and in embodiments may be from about 3 sccm to about 6 sccm. Hydrocarbon gas may be supplied at a flow rate from about 15 sccm to about 30 sccm, and in embodiments may be from about 20 sccm to about 25 sccm. Combined flow rate for oxygen and hydrocarbon gas may be from about 15 sccm to about 40 sccm, and in embodiments may be from about 20 sccm to about 35 sccm. In further embodiments, gases may be supplied at any suitable flow rate such that the ratio of oxygen to one or more hydrocarbon gases is from about 1:1 to about 1:10, in embodiments the ratio may be from about 1:4 to about 1:5.

Without being constrained by any particular theory, it is believed that application of an oxygen and hydrocarbon gas based plasma modifies the chemical properties of the cuff 72 by making the cuff 72 more hydrophilic, thereby promoting faster tissue ingrowth. In particular, plasma application results in oxidation of the materials of the cuff 72 by adding oxidized groups, such as hydroxyl, ether, ester, carbonyl, and the like on the surface thereof. Plasma-treated cuff 72 provides for enhanced tissue ingrowth properties including tissue penetration (i.e., depth), cell density, collagen production, and tissue adhesion strength.

The following Examples are being submitted to illustrate embodiments of the present disclosure. These Examples are intended to be illustrative only and are not intended to limit the scope of the present disclosure. Also, parts and percentages are by weight unless otherwise indicated. As used herein, "room temperature" or "ambient temperature" refers to a temperature of from about 20 ° C to about 25° C.

### EXAMPLES

### EXAMPLE 1 - Plasma Treatment of DACRON™ Catheter Cuff

A PJ Plasma Surface Treatment from AST Products of Billerica, MA was utilized to treat a DACRON™ cuff. Plasma was generated using oxygen supplied at a flow rate of about 5 sccm and methane supplied at a flow rate of about 25 sccm. The pressure within the plasma chamber was about 430 mTorr. Supplied power was about 100 W. The cuff was treated for about 5 minutes.

Plasma was also generated using oxygen supplied at a flow rate of about 10 sccm and methane supplied at a flow rate of about 25 sccm. The pressure within the plasma chamber was about 465 mTorr. Supplied power was about 120 W. The cuff was treated for about 5 minutes.

Plasma was further generated using oxygen supplied at a flow rate of about 5 sccm and methane supplied at a flow rate of about 20 sccm. The pressure within the plasma chamber was about 388 mTorr. Supplied power was about 100 W. The cuff was treated for about 5 minutes.

### EXAMPLE 2 - Subcutaneous Implantation of Hemodialysis Catheters

Catheters were subcutaneously implanted into rats with two (2) hemodialysis catheters (e.g., control and test catheters) per animal to compare tissue ingrowth of various catheter cuff materials. Each of the rats was implanted with a first catheter having a typical DACRON™ cuff used as a control and a second catheter including a plasma-treated cuff of Example 1. The catheters were implanted into the backs of the animals as shown in Fig. 3 for about seven (7) days (group I) and about twenty-eight (28) days (group II).

One group of the catheters were initially extracted after seven (7) days and a second group were extracted at twenty-eight (28) days following implantation. Transverse slices of the cuffs were obtained and observed under a microscope under a magnification of about 200 X.

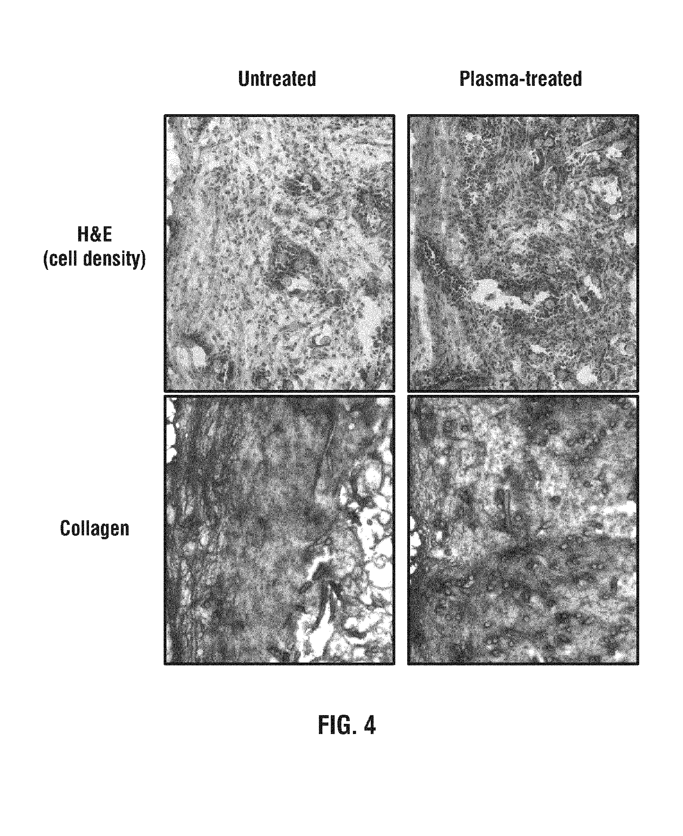

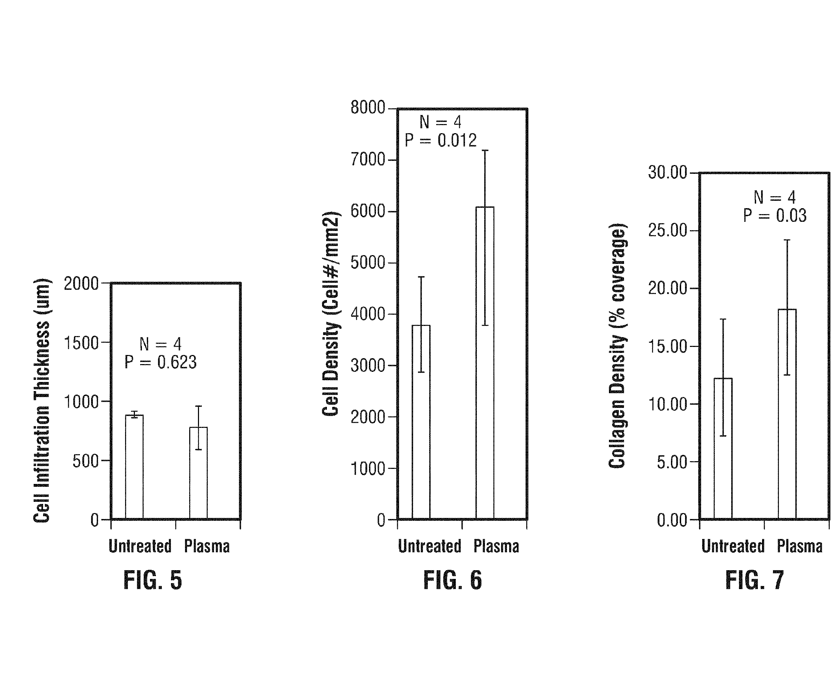

Fig. 4 illustrates images of the cuffs made from plasma treated DACRON™ of Example 1 and untreated DACRON™ material extracted after seven days (7) days. The observations of tissue infiltration are recorded in the bar graph of Fig. 5 illustrating tissue infiltration based on the thickness of the tissue in micrometers (µm). Both the untreated and treated DACRON™ cuffs exhibitied similar tissue infiltration after seven (7) days.

Cell density of the tissue ingrowth illustrated in Fig. 4 was also measured for each of the catheters. The observations of tissue ingrowth are recorded in the bar graph of Fig. 6 illustrating tissue inflitration based on cell density measured as cell count over area (cells/mm²). The plasma-treated DACRON™ cuff of Example 1 exhibited the greatest cell density after seven (7) days of implantation as compared to the untreated cuff as measured in terms of cell density and tissue thickness.

In addition to evaluating tissue thickness and cell density, collagen production in various cuffs was also compared. Fig. 7 illustrates tissue inflitration based on collagen production in terms of coverage percentage. Again, the plasma-treated DACRON™ cuff exhibited greater collagen density after seven (7) days of implantation as compared to the untreated cuff.

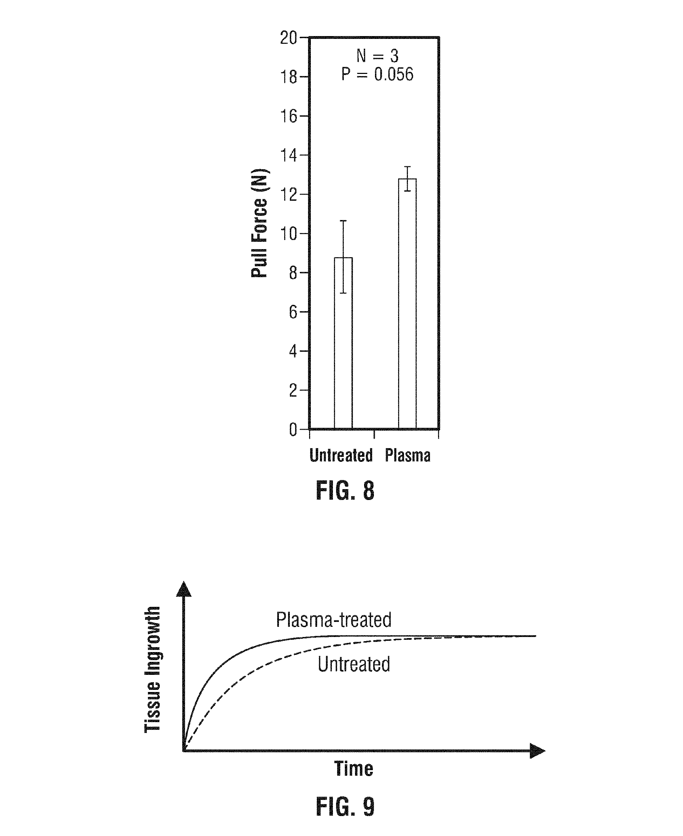

Effects of tissue ingrowth were also tested by measuring the tissue adhesion strength (e.g., pull force required to remove the catheter) of the cuffs. The observations of tissue adhesion are recorded in bar graph of Fig. 8, which illustrates tissue adhesion in terms of the pull force measured in Newtons (N). The plasma-treated DACRON™ cuff exhibited greater tissue adhesion after seven (7) days of implantation as compared to the untreated cuff.

The results of the bar graphs of Figs. 5-8 are summarized in a graph of Fig. 9 which illustrates the tissue ingrowth over time. As seen in the graph of Fig. 9, the plasma-treated DACRON™ cuffs reach the higher tissue ingrowth level at a faster rate than the untreated DACRON™ cuffs. Thus, plasma-treated DACRON™ cuffs were found to provide a greater amount of tissue growth attainable at a faster rate.

Although catheter cuffs made from DACRON™ have been discussed herein exclusively, it is believed that other materials, including, but not limited to, poly(lactic-co-glycolic acid), silicone rubber, and chitosan, would also benefit from plasma treatment with respect to enhanced tissue ingrowth properties.

Further, although the use of plasma treated cuffs has been discussed herein in association with a dual lumen hemodialysis catheters, it is envisioned that the advantages disclosed herein related to plasma treated cuffs are applicable to a variety of catheters, including single and multiple lumen catheters, that may benefit from the use of a cuff for long term catheterization. Accordingly, although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to those precise embodiments, and that various other changes and modifications may be effected therein by one skilled in the art without departing from the scope or spirit of the disclosure.

## Claims

1. A catheter comprising:
an elongated tubular body defining at least one lumen and a longitudinal axis, the tubular body having a distal end and a proximal end; and
a cuff disposed around the tubular body configured to contact tissue, the cuff formed from a plasma-treated material.

2. The catheter according to claim 1, wherein the catheter is a hemodialysis catheter configured for implantation into tissue.

3. The catheter according to any of claims 1 or 2, wherein the cuff is formed from an absorbable material.

4. The catheter according to any of claims 1 or 2, wherein the cuff is formed from a non-absorbable material.

5. The catheter according to claim 4, wherein the non-absorbable material is polyethylene terephthalate.

6. The catheter according to any of claims 1 or 2, wherein the cuff includes a plurality of layers, wherein at least one layer is formed from an absorbable material and at least one other layer is formed from a non-absorbable material.

7. The catheter according to any of the preceding claims, wherein the cuff is treated by a plasma of ionized oxygen and at least one hydrocarbon gas selected from the group consisting of methane, ethane, propane, butane, and combinations thereof.

8. A method for plasma-treating a catheter cuff, the method comprising:
supplying a mixture of oxygen and at least one hydrocarbon gas into a plasma chamber;
igniting the mixture to form a plasma; and
directing the plasma over a catheter cuff.

9. The method according to claim 8, wherein the pressure within the plasma chamber is maintained at about 50 mTorr to about 2000 mTorr and a ratio of oxygen to the at least one hydrocarbon gas is from about 1:4 to about 1:5

10. The method according to any of claims 8 or 9, wherein the step of directing occurs for a period of time of from about 10 seconds to about 20 minutes.

11. The method according to any of claims 8-10, wherein the cuff is formed from a material selected from the group consisting of poly(lactic-co-glycolic acid), polyethylene terephthalate, silicone rubber, chitosan, and combinations thereof.

12. The method of claim 8 wherein:
the at least one hydrocarbon gas is methane;
the mixture of oxygen and methane is supplied into the plasma chamber at a combined flow rate from about 15 sccm to about 40 sccm; and
the plasma is directed over the catheter cuff for about 1 minute to about 5 minutes.

13. The method according to claim 12, wherein the pressure within the plasma chamber is maintained at about 50 mTorr to about 2000 mTorr and a ratio of oxygen to methane is from about 1:4 to about 1:5

14. The method according to any of claims 12 or 13, wherein the step of igniting includes supplying electrical energy having an average power of from about 10 W to about 1000 W to the mixture of oxygen and methane.

15. The method according to any of claims 12-14, wherein the cuff is formed from polyethylene terephthalate.
